# EUROPÄISCHE PATENTSCHRIFT

(11) **EP 1 731 862 B1**
(45) Veröffentlichungstag und Bekanntmachung des Hinweises auf die Patenterteilung: **19.05.2010**
(21) Anmeldenummer: 06010605.1
(22) Anmeldetag: 23.05.2006
(51) Int. Cl.: F28D 15/00

(54) **Lichtquelle für die Endoskopie oder Mikroskopie**
Lightsource for endoscopy or microscopy
Source lumineuse pour l'endoscopie et la microscopie

(30) Priorität: 31.05.2005 DE 102005026662
(43) Veröffentlichungstag der Anmeldung: 13.12.2006
(73) Patentinhaber: Karl Storz GmbH & Co. KG, 78532 Tuttlingen (DE)
(72) Erfinder: Irion, Klaus M., Dr.-Ing., 78576 Liptingen (DE); Ehrhardt, André, 78573 Wurmlingen (DE); Simmen, Markus, 8603 Schwarzenbach (CH); Steiner, Jürg, CH-8400 Winterthur (CH)
(74) Vertreter: Heuckeroth, Volker

(56) Entgegenhaltungen:
- WO-A-99/16136
- WO-A-03/002909
- WO-A2-02/11640
- WO-A2-2004/011848

## Beschreibung

Die vorliegende Erfindung betrifft eine Lichtquelle für die Endoskopie oder Mikroskopie, mit einer Lampe und mit einem Kühlkörper, wobei der Kühlkörper thermisch mit der Lampe gekoppelt ist und wobei zumindest eine Heatpipe vorhanden ist, die mit dem Kühlkörper thermisch gekoppelt ist und die von der Lampe erzeugte und an den Kühlkörper abgegebenen Wärme ableitet.

Eine Lichtquelle mit diesen technischen Merkmalen ist z.B. aus der WO 2004/011848 A2 bekannt.

Solche Lichtquellen dienen dazu, z.B. ein mit Hilfe eines Endoskops zu beobachtenden Bereich zu beleuchten. Da der zu beobachtende Bereich normalerweise vollkommen verdunkelt ist und da das Licht meist über Lichtleiter geleitet wird, die nur einen geringen Querschnitt aufweisen, muss die Lichtquelle Licht mit einer hohen Leistung bzw. Leistungsdichte bereitstellen, um für eine ausreichende intrakorporale Beleuchtung zu sorgen. Dazu werden im Allgemeinen Lichtbogenlampen und spezifischer sog. Hochdruck-Xenon-Kurzbogenlampen verwendet.

Solche Lampen erzeugen neben einer hohen Lichtleistung gleichzeitig eine hohe Wärmeleistung. Bei einer zurzeit eingesetzten Xenon-Kurzbogenlampe mit einer elektrischen Gesamtleistung von 300 W werden weniger als 50 W als Lichtleistung, mehr als 250 W jedoch als Wärmeleistung abgegeben. Diese Wärme muss von der Lampe und aus der Lichtquelle abgeführt werden, da es ansonsten zu einem überhitzen und einer Beschädigung der Lichtquelle kommen kann.

Es ist nun Aufgabe der Erfindung, eine Lichtquelle zu beschreiben, bei der die Kühlung ohne einen aktiven Luftstrom vom Geräteinneren in die Umgebung erfolgt, der zu einer Kontamination eines sterilen Bereiches führen kann, sowie deutlich effizienter ist als bei bekannten Lichtquellen.

Erfindungsgemäß wird die Aufgabe dadurch gelöst, dass die Lampe in dem Kühlkörper angeordnet ist.

Bei einer Heatpipe handelt es sich um eine Vorrichtung, die aus einem abgeschlossenen Hohlkörper besteht, der im Allgemeinen aus einem hochwärmeleitfähigen Material, z.B. Kupfer oder Aluminium, hergestellt ist. An der Innenseite des Hohlkörpers ist ein kapillar wirksames, dochtartiges Material angeordnet. Der Hohlkörper ist ferner mit einer Flüssigkeit unter Eigendruck oder ggf. unter reduziertem Druck befüllt. Wird nun an irgendeinem Punkt der Oberfläche der Heatpipe Wärme zugeführt, beginnt die im Inneren der Heatpipe befindliche Flüssigkeit zu sieden und geht unter Wärmeenergieaufnahme in Dampf über. Dieser Dampf verteilt sich nun in dem Hohlkörper und kondensiert unter Wärmeabgabe an einer kälteren Stelle der Heatpipe. Das kapillar wirksame, dochtartige Material wiederum nimmt die kondensierte Flüssigkeit auf und transportiert diese zurück an die Stelle der Heatpipe, an der Wärme zugeführt wird. Hierdurch entsteht ein Kreislauf, durch den auf hocheffektive Weise Wärme von einer Stelle der Heatpipe an eine andere Stelle transportiert wird. Heatpipes können dabei eine um mehrere Größenordnungen bessere Wärmeleitfähigkeit als Kupfer aufweisen. Heatpipes haben ferner den Vorteil, dass sie vollkommen in sich abgeschlossene Systeme bilden, die den Wärmetransport ohne äußeren Einfluss, z.B. den Einsatz von Ventilatoren oder Pumpen, erreichen können.

Somit kann durch die Verwendung von zumindest einer Heatpipe Wärme von einer Lampe einer Lichtquelle für die Endoskopie oder Mikroskopie effektiv an einen anderen Ort, z.B. an die Außenseite eines Gehäuses, abgeführt werden, ohne dass der Einsatz von Ventilatoren notwendig wäre. Das Gehäuse kann dabei als vollständig abgeschlossene Einheit ausgelegt sein, so dass keine Kontaminationen aus dem Inneren austreten können.

Der Begriff "Kühlkörper" im Sinne der Erfindung bezeichnet jeden Körper, der in der Lage ist, Wärme von der Lampe aufzunehmen bzw. abzuleiten. Der Körper kann dabei eine beliebige Form aufweisen und aus einem beliebigen Material bestehen, insofern dieses in der Lage ist, die von der Lampe abgegebene Wärme aufzunehmen und an die Heatpipe weiterzuleiten. Als beispielhafte Materialien für den Kühlkörper sind stark wärmeleitende Metalle wie Kupfer oder Kupferlegierungen zu nennen.

Der Begriff "Lampe" im Sinne der Erfindung schließt alle dem Fachmann bekannten Lampentypen ein. Als bevorzugte Lampentypen sind dabei Lichtbogenlampen und insbesondere Xenon-Kurzbogenlampen zu nennen.

Der Begriff "thermisch gekoppelt" im Sinne der Erfindung schließt jede Art der Verbindung ein, die einen Wärmeübergang ermöglicht. Dies schließt sowohl eine direkte thermische Kopplung als auch eine indirekte thermische Kopplung über weitere Elemente ein.

In einer Ausgestaltung der Erfindung ist zwischen der Lampe und dem Kühlkörper eine elektrisch hoch isolierende Schicht angeordnet.

Der Kühlkörper besteht auf Grund der hohen benötigten Wärmeleitfähigkeit im Allgemeinen aus einem Metall, also einem elektrisch leitfähigen Material. Bei der Zündung einer Lampe müssen nun häufig sehr hohe Spannungen angelegt werden. Bei einer Xenon-Kurzbogenlampe z.B. kann die Zündspannung im Bereich von 20-30 kV liegen. Bei der Verwendung von so hohen Spannungen zur Zündung der Lampe kann es zu einem Spannungs- bzw. Ladungsdurchschlag auf den Kühlkörper kommen, der den Strom dann ggf. an das Gehäuse oder andere Bauteile weiterleitet. Dies kann zu einer Gefährdung eines Benutzers der Lichtquelle oder in der Umgebung befindlicher Geräte führen. Durch das Vorsehen einer elektrisch isolierenden Schicht zwischen der Lampe und dem Kühlkörper kann ein solches Durchschlagen verhindert werden.

In einer Ausgestaltung der oben genannten Maßnahme ist die elektrisch isolierende Schicht wärmeleitfähig.

Durch diese Maßnahme wird sichergestellt, dass die Ableitung der Wärme von der Lampe an die Heatpipe nicht durch die elektrisch leitfähige Schicht behindert wird.

In einer Ausgestaltung der zuvor genannten Maßnahme ist die elektrisch isolierende Schicht zur Körperschalldämmung ausgelegt.

Die zuvor beschriebenen Lichtquellen werden auch im sog. gepulsten Betrieb verwendet, das bedeutet, die Lampe wird in kurzen Abständen an- und ausgeschaltet. Beim Zünden der Lampe kommt es zu einer Geräuschentwicklung, was bei gepulstem Betrieb zu einem intermittierenden Geräusch von beträchtlicher Lautstärke führen kann. Die oben genannte Maßnahme führt dazu, dass eine solche Geräuschentwicklung reduziert wird.

In einer weiteren Ausgestaltung der Erfindung besteht die elektrisch isolierende Schicht aus einer Nitrid-Keramik, insbesondere aus einer Aluminiumnitrid-Keramik.

Nitrid-Keramiken und insbesondere Aluminiumnitrid-Keramiken haben den Vorteil, dass sie sowohl eine hohe Wärmeleitfähigkeit als auch eine hohe Durchschlagsfestigkeit gegenüber elektrischen Spannungen aufweisen. Hierdurch sorgen diese Materialien sowohl für eine gute Wärmeableitung als auch für eine hervorragende elektrische Isolierung.

In einer weiteren Ausgestaltung der zuvor genannten Maßnahme ist die elektrisch isolierende Schicht auf zumindest einer Oberfläche mit einer Kupferschicht versehen, insbesondere ist sie auf allen Oberflächen mit einer Kupferschicht versehen.

Diese Maßnahme optimiert den Wärmeübergang an der Oberfläche der elektrisch isolierenden Schicht weiter.

In einer weiteren Ausgestaltung der Erfindung ist die zumindest eine Heatpipe an einer von dem Kühlkörper entfernten Stelle mit einem wärmespeichernden bzw. wärmeabgebenden Element thermisch gekoppelt.

Die oben genannte Maßnahme erhöht die Effizienz der Wärmeabfuhr an der Stelle der Heatpipe, zu der die vom Kühlkörper abgeführte Wärme transportiert werden soll.

In einer Ausgestaltung der zuvor genannten Maßnahme ist das wärmespeichernde bzw. wärmeabgebende Element mit mehreren Heatpipes thermisch koppelbar. Insbesondere gehören die mehreren Heatpipes dabei zu verschiedenen Geräten.

Durch diese Maßnahme wird ein sog. Heatsink-Bus geschaffen, wobei mit einem einzigenwärmespeichernden bzw. wärmeabgebenden Element verschiedene Bauteile und ggf. sogar verschiedene Geräte gekühlt werden können.

In einer weiteren Ausgestaltung der oben genannten Maßnahme weist das wärmeabgebende Element Mikrostrukturierungen auf.

Durch diese Maßnahme wird die Oberfläche des wärmeabgebenden Elements erhöht. Dies wiederum macht die Wärmeabgabe an die Umgebung effizienter.

In einer weiteren Ausgestaltung der Erfindung ist die zumindest eine Heatpipe mit einem Träger für die Lichtquelle thermisch koppelbar.

Durch diese Maßnahme kann ein Träger für die Lichtquelle gleichzeitig zur Wärmeabfuhr verwendet werden.

In einer weiteren Ausgestaltung der Erfindung ist zumindest ein (interner) Ventilator vorhanden, der einen Luftstrom über eine Abstrahlfläche der Lampe erzeugt.

Die Wärmeabfuhr an den Seiten der Lampe, an denen sie mit dem Kühlkörper in Berührung steht, erfolgt im Allgemeinen sehr effizient. Die Abstrahlfläche kann allerdings nicht mit dem Kühlkörper verbunden sein, da diese zum Austritt des Lichtstrahls notwendigerweise offen sein muss. Es kann daher dort zu einer starken Wärmeentwicklung kommen, die nicht effizient abgeführt wird. Durch die oben genannte Maßnahme kann ein Hitzestau an der Abstrahlfläche verhindert werden.

In einer weiteren Ausgestaltung der zuvor genannten Maßnahme weist der Kühlkörper eine Flanke auf, die über die Abstrahlfläche der Lampe übersteht und die stromabwärts der Abstrahlfläche der Lampe in dem von dem Ventilator erzeugbaren Luftstrom angeordnet ist.

Durch diese Maßnahme wird die von der Lampe erwärmte Luft durch den Ventilator auf einen Teil des Kühlkörpers geleitet, der zumindest einen Teil der in dieser Luft aufgenommenen Wärme aufnimmt.

In einer weiteren Ausgestaltung der zuvor genannten Maßnahme weist die Flanke luftdurchströmbare Strukturen auf.

Durch diese Maßnahme kann die effektive Oberfläche, mit der der Kühlkörper von der durch die Lampe erwärmten Luft durchströmt wird, deutlich vergrößert werden, was den Wärmeübergang effizienter macht.

In einer weiteren Ausgestaltung der zuvor genannten Maßnahme verlaufen die luftdurchströmbaren Strukturen zumindest zum Teil entlang der zumindest einen Heatpipe.

Durch diese Maßnahme kann die von der Lampe erwärmte Luft durch den Kühlkörper entlang einer Heatpipe geführt werden, wodurch das Abführen der Wärme nochmals effizienter gemacht wird.

In einer weiteren Ausgestaltung der Erfindung liegt ferner ein Netzteil zur Versorgung der Lampe mit Strom vor, wobei das Netzteil zumindest eine Heatpipe zur Ableitung der entstehenden Wärme aufweist.

Durch diese Maßnahme kann eine vollständig gekapselte Vorrichtung geschaffen werden, die alle notwendigen Bauteile aufweist, wobei jedes Bauteil passiv gekühlt wird, so dass kein Luftstrom in oder aus einem Gehäuse der Lichtquelle notwendig ist.

Es versteht sich, dass die vorstehend genannten und die nachstehend noch zu nennenden Merkmale nicht nur in den angegebenen Kombinationen, sondern auch in anderen Kombinationen oder in Alleinstellung verwendbar sind, ohne den Rahmen der Erfindung zu verlassen.

Ausführungsbeispiele der Erfindung sind in den Zeichnungen dargestellt und werden in der nachfolgenden Beschreibung näher erläutert. Es zeigen:
- Fig. 1: eine Lichtquelle für die Endoskopie oder Mikroskopie,
- Fig. 2: eine Schrägansicht eines Lampenmoduls für eine Lichtquelle,
- Fig. 3: einen Teilschnitt durch das Lampenmodul von Fig. 2 entlang der Linie III-III, und
- Fig. 4: eine auf einem Träger angeordnete Lichtquelle.

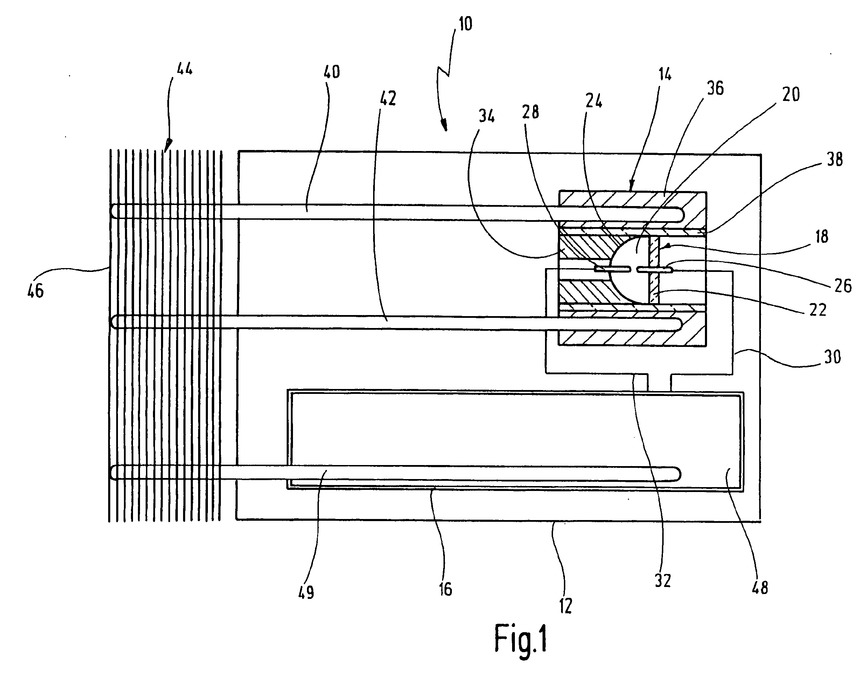

In Fig. 1 ist eine Lichtquelle in ihrer Gesamtheit mit der Bezugsziffer 10 bezeichnet.

Die Lichtquelle 10 weist ein Gehäuse 12 auf, in dem ein Lampenmodul 14 und ein Netzgerät 16 angeordnet sind.

Das Lampenmodul 14 weist eine Lampe, insbesondere Lichtbogenlampe, insbesondere in Form einer Xenon-Kurzbogenlampe 18, auf. Diese Xenon-Kurzbogenlampe 18 weist einen mit Xenon gefüllten Lampeninnenraum 20 auf, der an seiner Vorderseite durch eine Glasplatte 22 und an einer Rückseite durch einen Reflektor 24 begrenzt wird. An gegenüberliegenden Seiten sind in dem Lampeninnenraum 20 Elektroden 26 und 28 angeordnet, die über Leitungen 30 und 32 mit dem Netzgerät 16 verbunden sind.

Wird nun mittels des Netzgerätes 16 an die beiden Elektroden 26 und 28 eine ausreichend hohe Spannung angelegt, bildet sich zwischen den Elektroden 26 und 28 ein stehender Lichtbogen, wodurch Licht mit einem tageslichtähnlichen Spektrum erzeugt wird. Die zur Ausbildung eines Lichtbogens benötigte Spannung wird dabei im Allgemeinen als Zündspannung bezeichnet.

Die Xenon-Kurzbogenlampe 18 ist in einer Fassung 34 angeordnet, die sich innerhalb eines Kühlkörpers 36 befindet. Der Kühlkörper 36 besteht hierbei aus Kupfer und dient dazu, die durch die Xenon-Kurzbogenlampe 18 erzeugte Wärme möglichst effizient abzuleiten.

Zwischen dem Kühlkörper 36 und der Fassung 34 bzw. der Xenon-Kurzbogenlampe 18 ist außerdem eine elektrisch isolierende Schicht 38 angeordnet, die hier aus einer Aluminiumnitrid-Keramik besteht. Die Oberflächen der elektrisch isolierenden Schicht 38, die mit dem Kühlkörper 36 und der Fassung 34 bzw. der Xenon-Kurzbogenlampe 18 in Berührung stehen, sind durch ein Verfahren, das als Direct-Copper-Bonding (DCB) bezeichnet wird, mit einer Kupferschicht versehen, was den Wärmeübergang an den Oberflächen deutlich verbessert.

Bei diesem Verfahren werden auf den zu beschichtenden Oberflächen der Keramik mit Kuper(I)-Oxid versehene Kupferfolien angeordnet. Dieser Zusammenbau wird dann erwärmt, bis sich aus der Keramik und dem Kupfer(I)-Oxid ein Eutektikum bildet, wodurch nach Abkühlen eine feste Verbindung zwischen Keramik, Kupfer(I)-Oxid und Kupfer geschaffen wird.

Die elektrisch isolierende Schicht 38 verhindert einen Spannungsdurchschlag von der Xenon-Kurzbogenlampe 18 auf den Kühlkörper 36. Da die Kupferbeschichtung nur an der Oberfläche vorliegt, beeinträchtigt sie die elektrische Isolierung nicht.

Mit dem Kühlkörper 36 sind ferner Heatpipes 40 und 42 verbunden. In diesem Falle sind die Heatpipes 40 und 42 in Bohrungen in dem Kühlkörper 36 eingeführt, wobei zusätzlich noch eine Wärmeleitpaste zwischen den Heatpipes 40, 42 und dem Kühlkörper 36 eingebracht ist, um die Wärmeleitung weiter zu optimieren.

Die Heatpipes 40, 42 sind außerhalb des Gehäuses 12 der Lichtquelle 10 mit einem wärmeabgebenden Element 44 verbunden, das hier aus einer Vielzahl von Kühlrippen 46 besteht. In Betrieb nehmen nun die Heatpipes 40 und 42 die von der Xenon-Kurzbogenlampe 18 erzeugte und an den Kühlkörper 36 weitergeleitete Wärme auf und leiten diese an das wärmeabgebende Element 44, also nach außerhalb des Gehäuses 12 ab. Dies erfolgt vollständig passiv, so dass kein aktiver Luftstrom in das Gehäuse 12 hinein oder aus dem Gehäuse 12 heraus notwendig ist. Die Kühlrippen 46 weisen dabei eine ausreichend große Oberfläche auf, um ohne aktive Kühlung die von den Heatpipes 40, 42 abgeführte Wärme an die Umgebung abzugeben.

Das Netzteil 16 weist ferner eine Kühlplatte 48 auf, die die von dem Netzteil 16 erzeugte Wärme ableitet. Auf diese Kühlplatte 48 ist eine Heatpipe 49 aufgelötet, die die von der Kühlplatte 48 aufgenommene Wärme abführt. Die Heatpipe 49 ist ebenfalls mit dem wärmeabgebenden Element 44 verbunden, so dass die von dem Netzteil 16 erzeugte Wärme ebenfalls ohne aktive Kühlung aus dem Gehäuse 12 abgeführt werden kann.

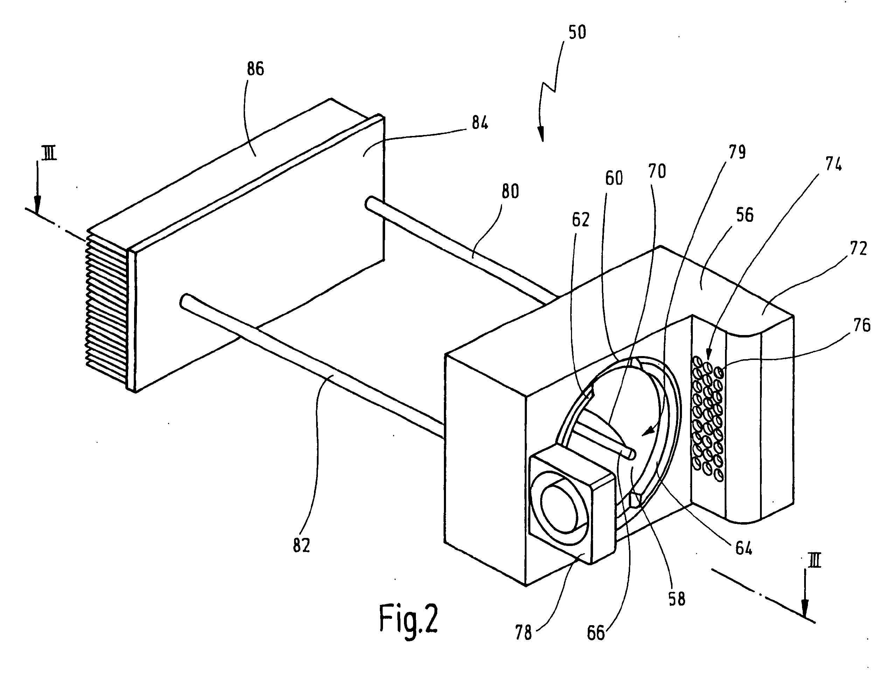

In Fig. 2 ist ein Lampenmodul für eine Lichtquelle für die Endoskopie oder Mikroskopie in seiner Gesamtheit mit der Bezugsziffer 50 bezeichnet.

Das Lampenmodul 50 weist einen Kühlkörper 56 auf, in dem eine Xenon-Kurzbogenlampe 58 angeordnet ist. Diese Xenon-Kurzbogenlampe wird mittels von Klammern 60 und 62 in dem Kühlkörper 56 gehalten. Ferner ist in dieser Darstellung eine Elektrode 66 sichtbar, die eine Elektrode des Elektrodenpaars der Xenon-Kurzbogenlampe 58 bildet, sowie eine Leitung 70, mit der die Elektrode 66 mit Strom versorgt werden kann.

Der Kühlkörper 56 weist ferner eine Flanke 72 auf, die nach vorne über die Xenon-Kurzbogenlampe 58 übersteht. In dieser Flanke 72 sind luftdurchströmbare Strukturen 74 in Form von Bohrungen 76 angeordnet.

Auf der der Flanke 72 gegenüberliegenden Seite des Kühlkörpers 56 ist ferner ein Ventilator 78 angeordnet, der einen Luftstrom über eine Abstrahlfläche 79 der Xenon-Kurzbogenlampe 58 in Richtung der Flanke 72 erzeugen kann.

An der Rückseite des Kühlkörpers treten zwei Heatpipes 80 und 82 aus, die den Kühlkörper mit einem wärmeabgebenden Element 84 verbinden, das wiederum eine Vielzahl von Kühlrippen 56 aufweist.

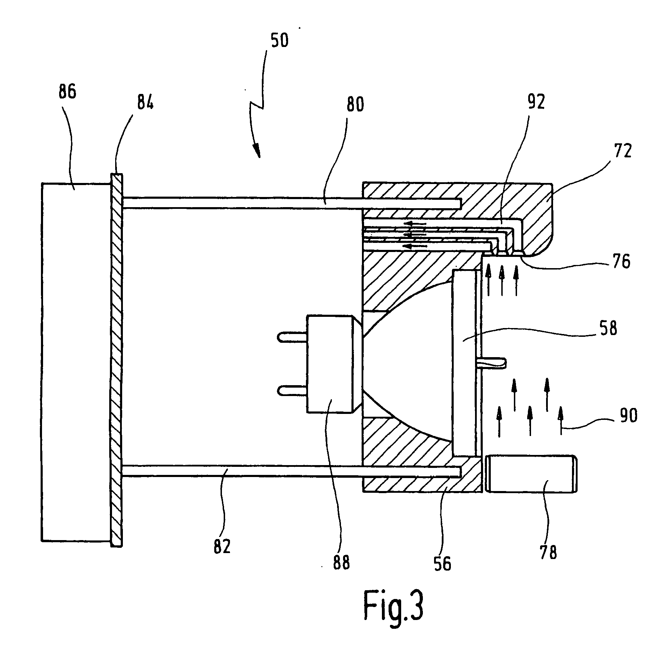

Aus Fig. 3 wird ersichtlich, dass diese Heatpipes 80 und 82 wiederum in Bohrungen in dem Kühlkörper 56 angeordnet sind.

Aus Fig. 3 wird ferner ersichtlich, dass die Xenon-Kurzbogenlampe 58 an ihrer Rückseite einen Stecker 88 aufweist, mit dem die diese mit Strom versorgt werden kann.

Während des Betriebs geht die an den Seiten der Xenon-Kurzbogenlampe 58 abgegebene Wärme auf den Kühlkörper 56 über und wird von diesem an die Heatpipes 80 und 82 übertragen. Diese Heatpipes 80 und 82 leiten die Wärme in Richtung des wärmeabgebenden Elements 84 ab, wobei das wärmeabgebende Element 84 die aufgenommene Wärme über die Kühlrippen 86 an die Umgebung abgibt.

Der Ventilator 78 kann nun einen Luftstrom in Richtung der Pfeile 90 erzeugen. Dieser Luftstrom bewegt sich entlang der Abstrahlfläche 79 der Xenon-Kurzbogenlampe 58 in Richtung der Flanke 72. Dabei nimmt der Luftstrom die von der Xenon-Kurzbogenlampe 58 an ihrer Abstrahlfläche 79 abgegebene Wärme auf und erwärmt sich. Der erwärmte Luftstrom tritt in die Bohrungen 76 ein, die sich in Form von Leitungen 92 in dem Kühlkörper 56 fortsetzen. Während des Durchströmens des Kühlkörpers 56 gibt der Luftstrom einen Teil der aufgenommenen Wärme bereits an den Kühlkörper 56 ab, wobei der Kühlkörper 56 diese dann über die Heatpipes 80 und 82 ableiten kann. Die Leitungen 92 verlaufen ferner über einen gewissen Abschnitt entlang der Heatpipe 80, wodurch die Wärmeabfuhr aus dem erwärmten Luftstrom in die Heatpipe 80 deutlich verbessert wird, so dass am hinteren Ende des Kühlkörpers 56 ein bereits deutlich abgekühlter Luftstrom austritt.

Somit wird die Bildung eines Hitzestaus an der Abstrahlfläche 79 der Xenon-Kurzbogenlampe 58 effektiv vermieden.

Bei dieser Ausführungsform wird zwar ein Ventilator verwendet, der durch diesen Ventilator erzeugte Luftstrom verläuft aber vollständig innerhalb des Gehäuses, so dass dadurch keine Kontamination einer sterilen Umgebung vorkommen kann.

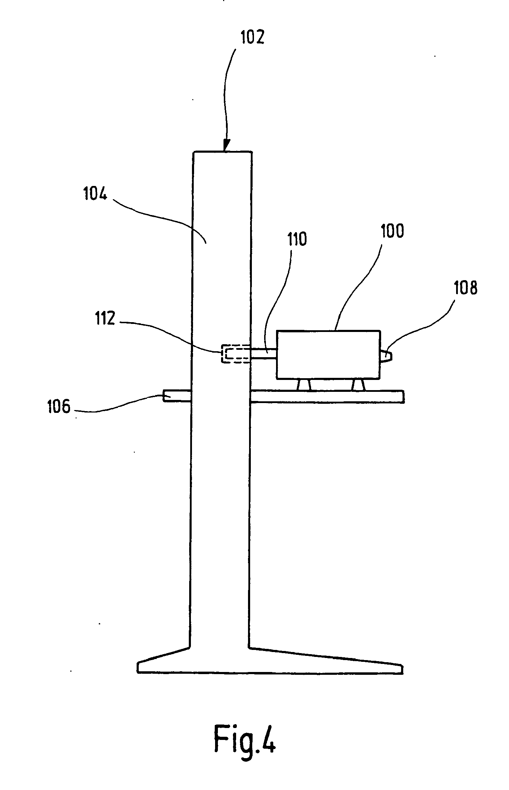

In Fig. 4 ist eine Lichtquelle in ihrer Gesamtheit mit der Bezugsziffer 100 bezeichnet.

Die Lichtquelle 100 ist auf einem Träger 102 angeordnet, der hier in Form eines Racks ausgebildet ist. Dieser Träger 102 weist zwei parallel zueinander angeordnete Seitenteile auf, von denen hier nur das Seitenteil 104 sichtbar ist. Zwischen diesen Seitenteilen ist eine Platte 106 angeordnet, auf der die Lichtquelle 100 angeordnet ist.

Die Lichtquelle 100 weist an ihrer Vorderseite einen Anschluss 108 zum Anschluss eines Lichtleiters auf. Mittels dieses Anschlusses 108 kann Licht der Lichtquelle 100 z.B. in den Lichtleiter eines Endoskops eingekoppelt und dazu verwendet werden, einen durch ein Endoskop zu beobachtenden Bereich zu beleuchten.

An der Rückseite der Lichtquelle 100 befindet sich das hintere Ende einer Heatpipe 110, die hier nicht sichtbar an ihrer Vorderseite mit einem Kühlkörper eines Lampenmoduls der Lichtquelle 100 verbunden ist und Wärme von diesem Lampenmodul an die Außenseite der Lichtquelle 100 ableitet.

Diese Heatpipe 110 ist zum Teil in eine Bohrung 112 des Seitenteils 104 eingeführt, wie dieses hier mit gestrichelten Linien dargestellt ist.

Zwischen der Heatpipe 110 und der Bohrung 112 im Seitenteil 104 ist ferner eine wärmeleitende Paste angeordnet, die den Wärmeübergang von der Heatpipe 110 auf das Seitenteil 104 erleichtert.

In dieser Ausführungsform wird die von der Heatpipe 110 aus der Lichtquelle 100 abgeführte Wärme an das Seitenteil 104 abgegeben. Es ist in diesem Fall kein separates wärmeableitendes Element mehr notwendig, da hier das Seitenteil 104 als wärmeableitendes Element dient. Dadurch, dass die Heatpipe 110 nur in das Seitenteil 104 eingesteckt ist, können modular auch andere Geräte, die ggf. ebenfalls mit Heatpipes ausgestattet sind, mit dem Träger 102 verwendet werden. Das Seitenteil 104 kann dabei auch die Heatpipes von mehreren Geräten aufnehmen und als sog. Heatsink-Bus wirken.

## Patentansprüche

1. Lichtquelle für die Endoskopie oder Mikroskopie, mit einer Lampe und mit einem Kühlkörper (36; 56), wobei der Kühlkörper (36; 56) thermisch mit der Lampe gekoppelt ist, und wobei zumindest eine Heatpipe (40, 42, 49; 80, 82; 110) vorhanden ist, die mit dem Kühlkörper (36; 56) thermisch gekoppelt ist und die von der Lampe erzeugte und an den Kühlkörper (36; 56) abgegebene Wärme ableitet, **dadurch gekennzeichnet, dass** die Lampe in dem Kühlkörper (36; 56) angeordnet ist.

2. Lichtquelle nach Anspruch 1, **dadurch gekennzeichnet, dass** zwischen der Lampe und dem Kühlkörper (36; 56) eine elektrisch hoch isolierende Schicht (38) angeordnet ist.

3. Lichtquelle nach Anspruch 2, **dadurch gekennzeichnet, dass** die elektrisch isolierende Schicht (38) wärmeleitfähig ist.

4. Lichtquelle nach Anspruch 2 oder 3, **dadurch gekennzeichnet, dass** die elektrisch isolierende Schicht (38) zur Körperschalldämmung ausgelegt ist.

5. Lichtquelle nach einem der Ansprüche 2 bis 4, **dadurch gekennzeichnet, dass** die elektrisch isolierende Schicht (38) aus einer Nitrid-Keramik besteht.

6. Lichtquelle nach Anspruch 5, **dadurch gekennzeichnet, dass** die elektrisch isolierende Schicht (38) aus Aluminiumnitrid-Keramik besteht.

7. Lichtquelle nach einem der Ansprüche 2 bis 6, **dadurch gekennzeichnet, dass** die elektrisch isolierende Schicht (38) auf zumindest einer Oberfläche mit einer Kupferschicht versehen ist.

8. Lichtquelle nach Anspruch 7, **dadurch gekennzeichnet, dass** die elektrisch isolierende Schicht (38) auf allen Oberflächen mit einer Kupferschicht versehen ist.

9. Lichtquelle nach einem der Ansprüche 1 bis 8, **dadurch gekennzeichnet, dass** die zumindest eine Heatpipe (40, 42, 49; 80, 82; 110) an einer von dem Kühlkörper (36; 56) entfernten Stelle mit einem wärmespeichernden bzw. wärmeabgebenden Element (44; 84) thermisch gekoppelt ist.

10. Lichtquelle nach Anspruch 9, **dadurch gekennzeichnet, dass** das wärmespeichernde bzw. wärmeabgebende Element (44; 84) mit mehreren Heatpipes (40, 42, 49; 80, 82; 110) thermisch koppelbar ist.

11. Lichtquelle nach Anspruch 10, **dadurch gekennzeichnet, dass** die mehreren Heatpipes (40, 42, 49; 80, 82; 110) zu verschiedenen Geräten gehören.

12. Lichtquelle nach einem der Ansprüche 9 bis 11, **dadurch gekennzeichnet, dass** das wärmeabgebende Element (44; 84) Mikrostrukturierungen aufweist.

13. Lichtquelle nach einem der Ansprüche 1 bis 12, **dadurch gekennzeichnet, dass** die zumindest eine Heatpipe (40, 42, 49; 80, 82; 110) mit einem Träger (102) für die Lichtquelle (10; 100) thermisch koppelbar ist.

14. Lichtquelle nach einem der Ansprüche 1 bis 13, **dadurch gekennzeichnet, dass** ferner zumindest ein Ventilator (78) vorhanden ist, der einen Luftstrom über eine Abstrahlfläche (79) der Lampe erzeugt.

15. Lichtquelle nach Anspruch 14, **dadurch gekennzeichnet, dass** der Kühlkörper (36; 56) eine Flanke (72) aufweist, die über die Abstrahlfläche (79) der Lampe übersteht und die stromabwärts der Abstrahlfläche (79) der Lampe in dem von dem Ventilator (78) erzeugbaren Luftstrom angeordnet ist.

16. Lichtquelle nach Anspruch 15, **dadurch gekennzeichnet, dass** die Flanke (72) luftdurchströmbare Strukturen (76, 92) aufweist.

17. Lichtquelle nach Anspruch 16, **dadurch gekennzeichnet, dass** die luftdurchströmbaren Strukturen (76, 92) zumindest zum Teil entlang der zumindest einen Heatpipe (40, 42, 49; 80, 82; 110) verlaufen.

18. Lichtquelle nach einem der Ansprüche 1 bis 17, **dadurch gekennzeichnet, dass** ferner ein Netzteil (16) zur Versorgung der Lampe mit Strom vorliegt, wobei das Netzteil (16) zumindest eine Heatpipe (40, 42, 49; 80, 82; 110) zur Ableitung entstehender Wärme aufweist.

## Claims

1. A light source for endoscopy or microscopy, comprising a lamp and a heat sink (36; 56), the heat sink (36; 56) being thermally coupled with the lamp, and there being at least one heat pipe (40, 42, 49; 80, 82; 110) which is thermally coupled with the heat sink (36; 56) and which carries off the heat that is generated by the lamp and transferred to the heat sink (36; 56), **characterized in that** the lamp is arranged in the heat sink (35; 56).

2. The light source of Claim 1, **characterized in that** there is an electrically highly insulating layer (38) between the lamp and the heat sink (36; 56).

3. The light source of Claim 2, **characterized in that** the electrically insulating layer (38) is thermally conductive.

4. The light source of Claim 2 or 3, **characterized in that** the electrically insulating layer (38) is designed so as to reduce structure-borne sound.

5. The light source of any one of Claims 2 through 4, **characterized in that** the electrically insulating layer (38) consists of a nitride ceramic.

6. The light source of Claim 5, **characterized in that** the electrically insulating layer (38) consists of aluminium nitride ceramic.

7. The light source of any one of Claims 2 through 6, **characterized in that** the electrically insulating layer (38) comprises a layer of copper on at least one surface.

8. The light source of Claim 7, **characterized in that** the electrically insulating layer (38) comprises a layer of copper on all surfaces.

9. The light source of any one of Claims 1 through 8, **characterized in that** the at least one heat pipe (40, 42, 49; 80, 82; 110) is thermally coupled to a heat-accumulating or heat-releasing element (44; 84) at a site remote from the heat sink (36; 56).

10. The light source of Claim 9, **characterized in that** the heat-accumulating or heat-releasing element (44; 84) can be thermally coupled to several heat pipes (40, 42, 49; 80, 82; 110).

11. The light source of Claim 10, **characterized in that** the several heat pipes (40, 42, 49; 80, 82; 110) belong to different devices.

12. The light source of any one of Claims 9 through 11, **characterized in that** the heat-releasing element (44; 84) comprises microstructuring.

13. The light source of any one of Claims 1 through 12, **characterized in that** the at least one heat pipe (40, 42, 49; 80, 82; 110) can be thermally coupled to a support (102) for the light source (10; 100).

14. The light source of any one of Claims 1 through 13, **characterized in that** there is also at least one fan (78), which generates a flow of air across a radiating surface (79) of the lamp.

15. The light source of Claim 14, **characterized in that** the heat sink (36; 56) comprises a flank (72), which projects beyond the radiating surface (79) of the lamp and which is sited downstream of the radiating surface (79) of the lamp in the flow of air that can be generated by the fan (78).

16. The light source of Claim 15, **characterized in that** the flank (72) comprises structures that air can pass through (76, 92).

17. The light source of Claim 16, **characterized in that** the structures that air can pass through (76, 92) run at least part way along the at least one heat pipe (40, 42, 49; 80, 82; 110).

18. The light source of any one of Claims 1 through 17, **characterized in that** there is also a power supply (16) to supply the lamp with power, the power supply (16) comprising at least one heat pipe (40, 42, 49; 80, 82; 110) to carry off the heat that is generated.

## Revendications

1. Source lumineuse pour l'endoscopie ou la microscopie, comportant une lampe et un dissipateur de chaleur (36 ; 56) couplé thermiquement à la lampe, et sachant qu'il est prévu au moins un caloduc (40, 42, 49 ; 80, 82 ; 110), qui est couplé thermiquement au dissipateur de chaleur (36 ; 56) et qui évacue la chaleur générée par la lampe et dégagée vers le dissipateur de chaleur (36 ; 56), **caractérisée en ce que** la lampe est agencée dans le dissipateur de chaleur (36 ; 56).

2. Source lumineuse selon la revendication 1, **caractérisée en ce qu'**une couche (38) très électro-isolante est disposée entre la lampe et le dissipateur de chaleur (36 ; 56).

3. Source lumineuse selon la revendication 2, **caractérisée en ce que** la couche (38) électro-isolante est une couche thermoconductrice.

4. Source lumineuse selon la revendication 2 ou 3, **caractérisée en ce que** la couche (38) électro-isolante est conçue pour l'insonorisation de la construction.

5. Source lumineuse selon l'une quelconque des revendications 2 à 4, **caractérisée en ce que** la couche (38) électro-isolante est réalisée en céramique à base de nitrure.

6. Source lumineuse selon la revendication 5, **caractérisée en ce que** la couche (38) électro-isolante est réalisée en céramique à base de nitrure d'aluminium.

7. Source lumineuse selon l'une quelconque des revendications 2 à 6, **caractérisée en ce que** la couche (38) électro-isolante est munie d'une couche de cuivre sur au moins une surface.

8. Source lumineuse selon la revendication 7, **caractérisée en ce que** la couche (38) électro-isolante est munie d'une couche de cuivre sur toutes les surfaces.

9. Source lumineuse selon l'une quelconque des revendications 1 à 8, **caractérisée en ce que** ledit au moins un caloduc (40, 42, 49 ; 80, 82 ; 110), à un emplacement éloigné du dissipateur de chaleur (36 ; 56), est couplé thermiquement à un élément (44 ; 84) thermo-accumulateur ou exothermique.

10. Source lumineuse selon la revendication 9, **caractérisée en ce que** l'élément (44 ; 84) thermo-accumulateur ou exothermique peut être couplé thermiquement à plusieurs caloducs (40, 42, 49 ; 80, 82 ; 110).

11. Source lumineuse selon la revendication 10, **caractérisée en ce que** la pluralité de caloducs (40, 42, 49 ; 80, 82 ; 110) appartiennent à différents appareils.

12. Source lumineuse selon l'une quelconque des revendications 9 à 11, **caractérisée en ce que** l'élément (44 ; 84) exothermique comporte des micro-structurations.

13. Source lumineuse selon l'une quelconque des revendications 1 à 12, **caractérisée en ce que** ledit au moins un caloduc (40, 42, 49 ; 80, 82 ; 110) peut être couplé thermiquement à un support (102) pour la source lumineuse (10 ; 100).

14. Source lumineuse selon l'une quelconque des revendications 1 à 13, **caractérisée en ce qu'**il est prévu, en outre, au moins un ventilateur (78) qui génère un flux d'air sur une surface radiante (79) de la lampe.

15. Source lumineuse selon la revendication 14, **caractérisée en ce que** le dissipateur de chaleur (36 ; 56) comporte un flanc (72), qui s'avance au-delà de la surface radiante (79) de la lampe et qui est disposé en aval de la surface radiante (79) de la lampe dans le flux d'air pouvant être généré par le ventilateur (78).

16. Source lumineuse selon la revendication 15, **caractérisée en ce que** le flanc (72) comporte des structures (76, 92) à travers lesquelles l'air peut circuler.

17. Source lumineuse selon la revendication 16, **caractérisée en ce que** les structures (76, 92), à travers lesquelles l'air peut circuler, s'étendent au moins en partie le long dudit au moins un caloduc (40, 42, 49 ; 80, 82 ; 110).

18. Source lumineuse selon l'une quelconque des revendications 1 à 17, **caractérisée en ce qu'**il est prévu, en outre, un bloc d'alimentation (16) pour l'alimentation en courant de la lampe, ledit bloc d'alimentation (16) comportant au moins un caloduc (40, 42, 49 ; 80, 82 ; 110) pour la dissipation de la chaleur générée.
